# EUROPÄISCHE PATENTSCHRIFT

(11) **EP 1 018 504 B1**
(45) Veröffentlichungstag und Bekanntmachung des Hinweises auf die Patenterteilung: **09.10.2002**
(21) Anmeldenummer: 99125968.0
(22) Anmeldetag: 27.12.1999
(51) Int. Cl.: C07C 68/06, C07C 69/96, C08G 64/30

(54) **Verfahren zur Herstellung von aliphatischen Oligocarbonatdiolen aus Dimethylcarbonat und aliphatischen Diolen**
Process for the preparation of aliphatic oligocarbonatediols from dimethyl carbonate and aliphatic diols
Procédé de préparation d'oligocarbonatediols aliphatiques à partir du carbonate de diméthyle et de diols aliphatiques

(30) Priorität: 09.01.1999 DE 19900554
(43) Veröffentlichungstag der Anmeldung: 12.07.2000
(73) Patentinhaber: BAYER AG, 51368 Leverkusen (DE)
(72) Erfinder: Langer, Reinhard, Dr., 47918 Tönisvorst (DE); Buysch, Hans-Josef, Dr., 47800 Krefeld (DE); Hovestadt, Wieland, Dr., 42799 Leichlingen (DE); Melchiors, Martin, Dr., 51373 Leverkusen (DE)

(56) Entgegenhaltungen:
- FR-A- 2 349 612
- FR-A- 2 373 570

## Beschreibung

Die vorliegende Erfindung betrifft ein verbessertes Verfahren zur Herstellung von aliphatischen Oligocarbonatdiolen aus nicht vicinalen Diolen durch Umesterung mit Dimethylcarbonat (DMC) in einer Gas-Flüssigkeits-Gegenstromapparatur und anschließende Abtrennung von Resten Methanol und Spuren Dimethylcarbonat in einer gasblasenerzeugenden Apparatur. Der im fertigen Oligocarbonat gelöste Katalysator kann entweder abgetrennt oder durch Zugabe von Komplexbildnern maskiert und im Oligocarbonat belassen werden.

Durch die erfindungsgemäße Verfahrensweise ist eine besonders wirtschaftliche Produktion von aliphatischen Oligocarbonatdiolen ausgehend von preiswertem Dimethylcarbonat möglich.

Aliphatische Oligocarbonate sind dem Fachmann als wichtige Zwischenprodukte, beispielsweise zur Herstellung von Kunststoffen, Lacken und Klebstoffen, z.B. durch Umsetzung mit Isocyanaten seit langem bekannt. Sie können prinzipiell aus nicht vicinalen Diolen durch Umsetzung mit Phosgen (DE-A 1 595 446), Bis-Chlorkohlensäureestern (DE-A 857 948), Diarylcarbonaten (DE-A 1 915 908), Dioxolanonen (DE-A 2 523 352) oder Dialkylcarbonaten (DE-A 2 555 805) hergestellt werden.

Von den oben genannten Carbonatquellen besitzt Diphenylcarbonat (DPC) eine besondere Bedeutung, da aus DPC aliphatische Oligocarbonatdiole von besonders hoher Qualität erzeugt werden können (z.B. US-A 3 544 524, EP-A 292 772 ,FR 2 349 612, FR-2 373 570).

DPC reagiert im Gegensatz zu allen anderen Carbonatquellen quantitativ mit aliphatischen OH-Funktionen, so dass nach dem Entfernen des entstandenen Phenols alle endständigen OH-Gruppen des Oligocarbonatgemisches für die Umsetzung mit Isocyanat-Gruppen zur Verfügung stehen. Ferner werden nur sehr geringe Konzentrationen an löslichem Katalysator benötigt, so dass dieser im Produkt verbleiben kann.

Zahlreiche Anmeldungen (z.B. die US-A 2 210 817, US-A 2 787 632, US-A 4 169 853, EP-A 364 052) befassen sich mit der Umsetzung von Dialkylcarbonaten mit aliphatischen Diolen.

Stand der Technik ist, aliphatische Diole zusammen mit dem Katalysator und dem Dialkylcarbonat vorzulegen und den entstehenden Alkohol (Ethanol, Butanol, Allylalkohol) aus dem Reaktionsgefäß so über eine Kolonne abzudestillieren, dass mitverdampftes Carbonat nicht entweichen kann. Abschließend wird die Umsetzung durch Anlegen von Vakuum zwecks Entfernung nicht umgesetzten Carbonates und Resten von Alkohol vervollständigt. Die Ansätze werden zu diesem Zweck üblicherweise nur beheizt und gerührt.

Die Verwendung von Dimethylcarbonat zur Herstellung von aliphatischen Oligocarbonatdiolen ist trotz seiner guten Zugänglichkeit erst seit kurzem bekannt, (US-A 5 171 830, EP-A 798 327, EP-A 798 328). Dies ist sicher durch den niedrigen Siedepunkt des DMC und die Existenz eines niedrig siedenden azeotropen Gemisches mit Methanol zu erklären. Beides erschwert den vollständigen Umsatz von DMC zu Oligocarbonatdiolen.

In den bisher bekannten Veröffentlichungen wird daher auch kein Verfahren mit hohen Raumzeitausbeuten und nahezu vollständigem Umsatz von DMC mit aliphatischen Diolen zu Oligocarbonatdiolen beschrieben. Der Zwangsanfall von DMC/Methanol-Gemischen unterschiedlicher Zusammensetzung schmälert die wirtschaftliche Attraktivität der beschriebenen Verfahren beträchtlich.

In der EP-A 798 327 wird entsprechend auch ein zweistufiges Verfahren beschrieben, in dem zuerst mit einem DMC-Überschuss ein Oligocarbonat erzeugt wird dessen endständige OH-Gruppen als Methoxycarbonat-Gruppen unzugänglich sind, und erst in einem zusätzlichen Schritt wird nach Zugabe eines weiteren Diols und nach insgesamt 36 Stunden Reaktionszeit das Oligocarbonatdiol erhalten.

Keine der Anmeldungen befasst sich mit der Realisierbarkeit der Methoden im technischen Maßstab.

Die durch lösliche Katalysatoren beschleunigte Umesterung von DMC mit höhersiedenden aliphatischen Alkoholen mit nur einer OH-Funktion (Monoole) in einer Gas-Flüssigkeits-Gegenstromkolonne wird in der DE-A 19 623 508 beschrieben. Zur Erreichung hoher DMC-Umsätze (89-99 %) mussten relativ niedrige Umsätze an hochsiedendem Alkohol (35 bis 58 %) in Kauf genommen werden. Dies legt nahe, dass diese Technik zum Aufbau hochmolekularer Oligoester ungeeignet ist.

Wünschenswert ist ein technisch in großen Einheiten umsetzbares Verfahren, das erlaubt, DMC mit guten Raumzeitausbeuten und hohem Umsatzgrad in einfachen Apparaturen mit aliphatischen Diolen zu Oligocarbonatdiolen umzusetzen.

Es wurde nun gefunden, dass man die Herstellung von aliphatischen Oligocarbonatdiolen mit einem Verkappungsgrad der endständigen OH-Gruppen durch Methoxycarbonyl-Gruppen kleiner als 1 % durch Umsetzung von Dimethylcarbonat mit aliphatischen Diolen mit einem Umsatzgrad des eingesetzten Dimethylcarbonates größer 98 % erreichen kann, indem die Umsetzung von Dimethylcarbonat und aliphatischen Diolen beschleunigt durch lösliche Katalysatoren in einer Gas-Flüssigkeits-Gegenstromapparatur durchgeführt wird, gefolgt von einer Abtrennung von Resten Methanol und Spuren Dimethylcarbonat in einer Apparatur die Gasblasen im Oligocarbonat erzeugt.

Die Erfindung betrifft demnach ein Verfahren zur Herstellung aliphatischer Oligocarbonate mit einem Verkappungsgrad der endständigen OH-Gruppen durch Methoxycarbonyl-Gruppen kleiner als 5 %, bevorzugt kleiner als 1 % durch Umsetzung von Dimethylcarbonat mit aliphatischen Diolen mit einem Umsatzgrad des eingesetzten Dimethylcarbonates größer 80 %, bevorzugt größer 90 %, besonders bevorzugt größer 95 %, insbesondere größer 98 %, dadurch gekennzeichnet, dass die Umsetzung von Dimethylcarbonat und aliphatischen Diolen beschleunigt durch lösliche Katalysatoren in einer Gas-Flüssigkeits-Gegenstromapparatur durchgeführt wird, gefolgt von einer Oligomerisation unter Abspaltung von Methanol und Spuren Dimethylcarbonat in einer Apparatur die Gasblasen im Oligocarbonat erzeugt.

Als Gas-Flüssigkeits-Gegenstromapparatur können verwendet werden: z.B. eine Glockenbodenkolonne mit 2 bis 20 Böden, gegebenenfalls mit größeren Flüssigkeits-hold-up, wie sie Stand der Technik sind; Blasensäulen-Kaskaden mit 2 bis 8, bevorzugt 2 bis 8, bevorzugt 2 bis 4 Blasensäulen, wobei mehrere Blasensäulen ohne Einbauten zur Verhinderung von Rückvermischungen durch eine Kolonne mit Einbauten ersetzt werden können.

Ferner kann als Gas-Flüssigkeits-Gegenstromapparatur eine Kesselkaskade mit 2 bis 6, bevorzugt 2 bis 3 Kesseln verwendet werden, wobei die Kessel bevorzugt mit Begasungsrührern ausgestattet sind.

In der Gegenstromapparatur wird das erfindungsgemäße Verfahren bei Temperaturen zwischen 100 und 250°C, bevorzugt zwischen 150 und 200°C und bei Drucken zwischen 0,8 und 8, bevorzugt zwischen 1 und 4 bar durchgeführt.

Als lösliche Katalysatoren können prinzipiell alle bekannten Umesterungskatalysatoren eingesetzt werden, insbesondere die Hydroxide der Alkali- und Erdalkalimetalle und die Metallalkoholate von aliphatischen Alkoholen mit 1-8 C-Atomen von Metallen der I., II., III. und IV. Hauptgruppe, der II. und IV. Nebengruppe oder aus der Gruppe der Seltenerden des Periodensystems der Elemente nach Mendelejew eingesetzt werden. Bevorzugt werden Natrium- und Kaliumalkoholate oder Titan- und Zirkoniumalkoholate verwendet, wobei Titan- und Zirkoniumtetraalkoholat bevorzugt bei Diolen, welche Esterfunktionen enthalten, eingesetzt wird.

Als Umesterungskatalysatoren seien bevorzugt genannt: Natriummethylat, Kaliummethylat, Natriumhydroxid, Kaliumhydroxid, Titan-tetra-iso-propylat und Zirkonium-tetra-isopropylat.

Im erfindungsgemäßen Verfahren werden Katalysatorkonzentrationen, angegeben in Gewichtsprozent Metall, bezogen auf eingesetztes aliphatisches Diol, zwischen 0,001 und 1 %, bevorzugt zwischen 0,005 und 0,5 %, besonders bevorzugt zwischen 0,02 und 0,2 %, eingesetzt.

Als Apparatur, die Gasblasen im Oligocarbonat erzeugt, kann eine Blasensäulen-Kaskade oder eine Kesselkaskade bevorzugt mit Begasungsrührern eingesetzt werden. Die Gasblasen erzeugende Vorrichtung kann auch in der Gas-/Flüssigkeits-Gegenstromapparatur integriert sein.

In der Apparatur, die Gasblasen im Oligocarbonat erzeugt, wird bei Temperaturen zwischen 150 und 250°C, bevorzugt zwischen 170 und 220°C, und bei Drucken zwischen 0,01 und 1, bevorzugt zwischen 0,05 und 0,5 bar, gearbeitet.

Für Produkte mit mittlerer und kleiner Jahrestonnage wird das erfindungsgemäße Verfahren, bevorzugt als Semibatch-Verfahren, durchgeführt mit einer großen Blasensäule oder einem großen Kessel, bevorzugt mit Begasungsrührer, in den das Dimethylcarbonat dosiert wird und in dem abschließend die Demethoxylierung stattfindet, sowie mit aufgesetzter Glockenbodenkolonne, kleiner Kesselkaskade oder Blasensäulenkaskade, in der die Gegenstromumesterung stattfindet, wobei das Flüssigkeitsvolumen des Gegenstromanlagenteils zwischen 0,5 bis 50 %, bevorzugt zwischen 1 und 25 %, besonders bevorzugt zwischen 2 und 12 % des Kesselvolumens des großen Kessels beträgt.

Das erfindungsgemäßen Verfahren erlaubt Oligocarbonatdiole der Formel (1) mit einer Kohlenstoffzahl von 30 bis 300, bevorzugt von 60 bis 200, besonders bevorzugt von 100 bis 150 zu erzeugen.

In Formel (I) 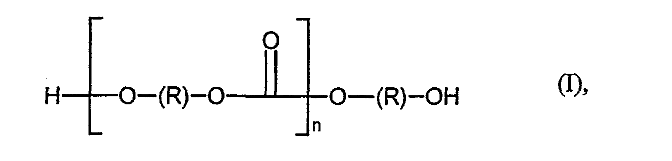 steht
- (R): für aliphatische Reste mit einer Kohlenstoffzahl von 3 bis 50, bevorzugt von 4 bis 40, besonders bevorzugt von 6 bis 30,
die gegebenenfalls Ester, Ether, Amid und Nitril-Funktionen enthalten können und
- n: für eine ganze Zahl von 2 bis 30, vorzugsweise 3 bis 20, besonders bevorzugt 3 bis 15.

Aliphatische Diole, die bevorzugt zur Herstellung der Oligocarbonatdiole der Formel (I) eingesetzt werden, sind Pentamethylendiol, Hexamethylendiol, Neopentylglykol, Cyclohexandimethanol und die Additionsprodukte aus Caprolacton mit den oben genannten Alkoholen (= Esterdiole) und Mischungen all dieser Diole. Bevorzugt werden Diole mit Ester-Funktionen, wie sie durch den Einsatz von Caprolacton und Hexamethylendiol erhalten werden, eingesetzt. Es können auch Mischungen von Caprolacton mit den genannten Diolen eingesetzt werden, wobei die daraus resultierenden Esterdiole in situ bilden.

Wichtiger Bestandteil des erfindungsgemäßen Verfahrens sind die Gasblasen in der gasblasenerzeugenden Apparatur. Diese Gasblasen werden durch Einleiten von inerten Gasen wie Stickstoff, Argon, Methan, Ethan, Propan, Butan, Dimethylether, trockenem Erdgas oder trockenem Wasserstoff in die gasblasenerzeugende Apparatur erzeugt, wobei der das Oligocarbonat verlassende, Methanol und Dimethylcarbonat haltige Gasstrom teilweise zur Sättigung erneut dem Oligocarbonat zugeführt werden kann. Diese Gasblasen können ferner durch Einleiten von inerten leichtsiedenden Flüssigkeiten wie Pentan, Cyclopentan, Hexan, Cyclohexan, Petrolether, Diethylether oder Methyl-tert.-Butylether erzeugt werden, wobei die Stoffe flüssig oder gasförmig eingeleitet werden können und der das Oligocarbonat verlassende Methanol und Dimethylcarbonat haltige Gasstrom teilweise zur Sättigung erneut dem Oligocarbonat zugeführt werden kann.

Die Stoffe zur Erzeugung von Gasblasen werden bevorzugt mittels Ringdüsen oder Begasungsrührern in das Oligocarbonat eingebracht, wobei Ringdüsen vorzugsweise bei Blasensäulen und Begasungsrührer bei Rührkesseln eingesetzt werden.

Eine technische Anlage zur Produktion von Oligocarbonatdiolen kann beispielsweise wie folgt aufgebaut sein: Ein 10 m3-Kessel mit einem bis zum Boden reichenden Einleitungsrohr für DMC und einem Begasungsrührer kann wahlweise über einen Kondensator mit einem Auffanggefäß für Leichtsieder an eine Vakuumpumpe oder an eine 4-stufige Blasensäulen-Kaskade mit Druckhalteventil, nachfolgendem Kondensator und Auffanggefäß für Leichtsieder angeschlossen werden. Der Kessel und jede der Blasensäulen ist beheizbar. Jede Blasensäule hat ein Flüssigkeitsvolumen von 100 L. Die Blasensäulen sind derart in Reihe geschaltet, dass der Gasstrom aus dem Kessel und das Gemisch aus aliphatischem Diol und Katalysator im Gegenstrom geführt werden. Der Kessel kann bei bis zu 3 bar Überdruck mit der Blasensäulenkaskade oder bei bis zu 50 mbar Absolutdruck mit der Vakuumanlage betrieben werden. Der Begasungsrührer des Reaktionskessels kann wahlweise mit Frischgas oder mit Gas aus dem Kesselraum oder mit Gemischen dieser Gase betrieben werden.

Eine Semibatch-Produktion kann wie folgt durchgeführt werden: Kessel und Blasensäulen stehen unter Stickstoff und werden auf Reaktionstemperatur aufgeheizt, die Dosierung des Diol-Katalysatorgemisches wird gestartet. Das Gemisch wird der obersten Blasensäule zugeführt, von der es nach deren vollständiger Füllung weiter zur nächsten und schließlich in den Kessel fließt. Wenn der Kessel soweit gefüllt ist, dass der Begasungsrührer in die Flüssigkeit eintaucht, wird die DMC-Dosierung gestartet, der Begasungsrührer in Betrieb genommen und das Druckhalteventil auf den gewünschten Druck eingestellt. Das den Reaktor verlassende Kondensat bestehend aus Methanol und wenig DMC wird auf seine Zusammensetzung untersucht und die einzudosierende DMC-Menge um die nicht aufgenommene korrigiert. Nachdem die berechnete Diol-DMC-Menge zugepumpt worden ist, wird die Verbindung zwischen Kessel und Blasensäulenkaskade geschlossen und die zur Vakuumpumpe geöffnet. Durch Anlegen eines leichten Vakuums wird begonnen, Leichtsieder abzudestillieren. Dabei wird der Begasungsrührer mit Kesselgas betrieben. Wenn die Destillationsgeschwindigkeit nachlässt, wird das Vakuum angehoben, bis ein Vakuum von 150 mbar erreicht wird. Bei nachlassender Destillationsgeschwindigkeit wird die Gaszuspeisung für den Begasungsrührer auf eine einzutragende inerte Verbindung umgestellt, z.B. Stickstoff. Von Zeit zu Zeit kann der Kesselinhalt auf Reste eingebauten Methanols untersucht werden. Nach Unterschreiten eines festgelegten Verkappungsgrades wird mit Stickstoff belüftet und der Kesselinhalt abgekühlt, gegebenenfalls wird ein Komplexbildner für den eingesetzten Katalysator eingerührt, bzw. der saure Katalysator neutralisiert oder der Kesselinhalt einer Aufarbeitung zur Abtrennung von störendem Katalysator zugeführt.

Die beschriebene Semibatch-Fahrweise ist nur ein Beispiel und nicht einschränkend zu verstehen. Dem Durchschnittsfachmann ist prinzipiell bekannt, wie solche Prozesse vollkontinuierlich durchzuführen sind.

Das erfindungsgemäße Verfahren erlaubt die Produktion von hochwertigen Oligocarbonatdiolen aus DMC mit guten Raumzeitausbeuten, hohen Umsätzen des DMCs und geringem Verkappungsgrad der endständigen OH-Gruppen.

Die nach dem erfindungsgemäßen Verfahren hergestellten Oligocarbonate können z.B. zur Herstellung von Kunststoffen, Fasern, Klebstoffen oder Beschichtungen verwendet werden. Sie können weiterhin als Bindemittel, Bindemittelbestandteil und/oder Reaktivverdünner in lösemittelfreien oder lösemittelarmen Polyurethanbeschichtungen verwendet werden. Sie eignen sich als Baustein für feuchtigkeitshärtende Beschichtungen, als Baustein und/oder Bindemittelbestandteil in lösemittelhaltigen oder wässrigen Polyurethanbeschichtungen. Sie können weiterhin als Baustein für freie NCO-Gruppen enthaltende Polyurethanprepolymere eingesetzt werden ebenso wie als Baustein in Polyurethandispersionen.

Die nach dem erfindungsgemäßen Verfahren hergestellten Oligocarbonatdiole können auch zur Herstellung thermoplastischer Kunststoffe wie aliphatischer und/oder aromatischer Polycarbonate, thermoplastischer Polyurethane usw. eingesetzt werden.

### Beispiele

Die %-Angaben für die Zusammensetzungen der erhaltenen Destillate sind immer mol-%, während %-Gehalte für Verbindungen in den Sumpfphasen und Katalysatorgehalte der aliphatischen Diole immer Gew.-% sind.
NL/h = liter/Stunde bei p = 1 bar, T = 20°C

### Beispiel 1

### Kaliumalkoholat katalysierte Umesterung von DMC mit Hexamethylendiol in einer kontinuierlich betriebenen Umesterungskolonne

Die Apparatur bestand aus einer 20-bödigen ölthermostatisierten Glockenbodenkolonne mit einem Innendurchmesser von 5 cm und einem Flüssigkeits-hold-up von ca. 850 ml. Die Kolonne hatte am Gasausgang einen thermostatisierten Dephlegmator und am Flüssigkeitsausgang einen ölbeheizten Naturumlaufverdampfer mit einem Flüssigkeitsinhalt von ca. 70 ml.

Die Kolonne wurde mit einem Strom aus 120°C warmem Wärmeträgeröl thermostatisiert.

Der Dephlegmator wurde mit 80°C und der Umlaufverdampfer mit 180°C warmem Öl beheizt.

Die Kolonne wurde bei Umgebungsdruck betrieben.

Mittels einer Dosierpumpe wurden dem obersten Kolonnenboden pro Stunde 640 ml einer 120°C warmen Mischung aus Hexamethylendiol mit 0,28 % Kaliumhydroxid zugepumpt. Zwischen Kolonne und Umlaufverdampfer wurde gleichzeitig ein 120°C warmer Gasstrom aus Dimethylcarbonat eingeleitet. Dieser Gasstrom wurde durch Verdampfung von 330 ml Dimethylcarbonat pro Stunde erzeugt.

Der Kolonne wurde damit ein Molverhältnis von DMC zu Hexamethylendiol von 1 zu 1,25 zugeführt.

Der Gasstrom, der den Dephlegmator verlässt, wurde kondensiert und aufgefangen, pro Stunde fielen dort 275 bis 285 ml/h Flüssigkeit an. Die gaschromatographische Analyse zeigte, dass diese Flüssigkeit zu 99,986 % aus Methanol und 0,014 % aus Hexamethylendiol bestand. Der Dimethylcarbonat-Gehalt lag unter der Nachweisgrenze von 0,01 %.

Am Verdampfer wurden pro Stunde von ca. 712 g einer farblosen flüssigen Substanz abgezogen. Die gaschromatographische Analyse dieser Mischung zeigte, dass ca. 4 % nicht umgesetztes Methanol, 0,7 % Dimethylcarbonat und 7,4 % unumgesetztes Hexamethylendiol enthalten waren.

### Beispiel 2

### Natriumalkoholat katalysierte Umesterung von DMC mit Hexamethylendiol in einer kontinuierlich betriebenen Umesterungskolonne

Der Versuch aus Beispiel 1 wurde wiederholt mit dem Unterschied, dass anstatt Kaliumhydroxid 0,14 % Natriumhydroxid im Hexamethylendiol-Strom enthalten waren.

Der Gasstrom, der den Dephlegmator verlässt, wurde kondensiert und aufgefangen, pro Stunde fielen dort 276 bis 280 ml/h Flüssigkeit an. Die gaschromatographische Analyse zeigte, dass diese Flüssigkeit zu 99,98 % aus Methanol und 0,02 % aus Hexamethylendiol bestand. Der Dimethylcarbonat-Gehalt lag unter der Nachweisgrenze von 0,01 %.

Der Verdampfer wurde pro Stunde von ca. 710 g einer farblosen flüssigen Substanz verlassen. Die gaschromatographische Analyse dieser Mischung zeigte, dass ca. 4,5 % ungebundenes Methanol, 0,7 % Dimethylcarbonat und 8,0 % unumgesetztes Hexamethylendiol enthalten waren.

### Beispiel 3

### Natriumalkoholat katalysierte Umesterung von DMC mit Hexamethylendiol mit sehr großer Raumzeitausbeute in einer kontinuierlich betriebenen Umesterungskolonne

Der Versuch aus Beispiel 2 wurde wiederholt mit dem Unterschied, dass die dreifache Menge an DMC und Hexandiol-Natriumhydroxid-Gemisch in die Kolonne gefahren wurde.

Der Gasstrom, der den Dephlegmator verlässt, wurde kondensiert und aufgefangen, pro Stunde fielen dort ca. 840 ml/h Flüssigkeit an. Die gaschromatographische Analyse zeigte, dass diese Flüssigkeit zu 99,928 % aus Methanol und 0,045 % aus Hexamethylendiol bestand. Der Dimethylcarbonat-Gehalt stieg über die Nachweisgrenze und betrug 0,027 %. Der Verdampfer wurde pro Stunde von ca. 2150 g einer farblosen flüssigen Substanz verlassen. Die gaschromatographische Analyse dieser Mischung zeigte, dass ca. 5,9 % ungebundenes Methanol, 1,0 % Dimethylcarbonat und 9,5 % unumgesetztes Hexamethylendiol enthalten waren.

### Beispiel 4

### Natriumalkoholat katalysierte Umesterung von DMC mit einer äquimolaren Mischung aus Hexamethylendiol, Neopentylglykol und Cyclohexandimethanol in einer kontinuierlich betriebenen Umesterungskolonne

Die Versuchsapparatur aus Beispiel 1 wurde auf 140°C aufgeheizt, der Dephlegmator hatte eine Temperatur von 80°C und der Umlaufverdampfer eine von 220°C. Auf den obersten Kolonnenboden wurden pro Stunde 645 ml einer 140°C warmen äquimolaren Mischung aus Hexamethylendiol, Neopentylglykol und Cyclohexandimethanol gepumpt. Die Mischung enthielt 0,1 % Natriumhydroxid. Gleichzeitig wurde ein 140°C warmer Gasstrom erzeugt durch eine stündliche Verdampfung von 205 ml DMC eingeleitet. Das molare Verhältnis von Diol zu DMC betrug damit ca. 2,1 zu 1.

Der Gasstrom, der den Dephlegmator verlässt, wurde kondensiert und aufgefangen, pro Stunde fielen dort ca. 190 ml/h Flüssigkeit an. Die gaschromatographische Analyse zeigte, dass diese Flüssigkeit folgende Zusammensetzung hatte: 99,84 % Methanol, 0,04 % DMC, 0,10 % Neopentylglykol und 0,02 % Cyclohexandimethanol. Der Hexamethylendiol-Gehalt des Destillates lag unter der Nachweisgrenze.

Der Verdampfer wurde pro Stunde von ca. 660 g einer farblosen flüssigen Substanz verlassen. Die gaschromatographische Analyse dieser Mischung zeigte, dass ca. 0,7 % ungebundenes Methanol, 0,04 % Dimethylcarbonat, 3,8 % unumgesetztes Hexamethylendiol, 4,4 % unumgesetztes Neopentylglykol, 3,5 % unumgesetztes Cyclohexandimethanol und 3,6 % Neopentylglykolcarbonat enthalten waren.

### Beispiel 5

### Natriumalkoholat katalysierte Umesterung von DMC mit Hexamethylendiol in einem Semibatch betriebenen Kessel mit 10-bödiger Gegenstromkolonne

Die Apparatur bestand aus einem 5 L-Planschlifftopf mit Blattrührer, Wellenbrechern, Einleitrohr, einer 10 bödigen ölbeheizten Glockenbodenkolonne mit Dephlegmator und einem Totalkondensator für das die Kolonne verlassende Gas. Die Kolonne hatte einen Flüssigkeits-hold-up von ca. 170 ml.

Kolonne und Kessel wurden auf 160°C aufgeheizt und mit Stickstoff vollständig inertisiert. Der Dephlegmator wurde auf 80°C thermostatisiert.

Dann wurde begonnen, pro Stunde 940 ml (9,2 mol) 160°C warmes Hexamethylendiol mit 0,14 % Natriumhydroxid auf den obersten Boden zu pumpen.

Nachdem 860 ml (6,92 mol) Hexamethylendiol-Natriumhydroxid-Mischung zugepumpt worden waren, wurde der Rührer in Betrieb genommen und begonnen, zusätzlich 780 ml (9,2 mol) Dimethylcarbonat pro Stunde durch das Einleitrohr in die Flüssigkeit am Boden des Kessels zu fördern.

Nach drei Stunden wurde die Zudosierung eingestellt.

Insgesamt 1475 g Destillat waren in dieser Zeit entstanden, das sich wie folgt zusammensetzt: 99,2 % Methanol, 0,6 % Dimethylcarbonat und 0,2 % Hexamethylendiol.

Im Sumpfkolben befanden sich 4456 g einer farblosen Flüssigkeit, die 3,4 % Methanol, 1,1 % DMC und 4,2 % unumgesetztes Hexamethylendiol enthält.

Anschließend wurden bei 120°C und 20 bis 40 mbar flüchtige Bestandteile durch die Kolonne abgezogen. Nach 3 Stunden war der Methanolgehalt auf 0,1 % gesunken. Dimethylcarbonat war nicht mehr nachweisbar. Daraufhin wurde begonnen, bei Normaldruck 40 NL Stickstoff pro Stunde durch das Einleitrohr einzublasen. Die Kolonne war hierzu durch ein Abgasrohr ersetzt worden. In 3 Stunden fiel der Methanolgehalt auf 400 ppm, worauf 2 Stunden bei 140°C 60 NL Stickstoff pro Stunde eingeleitet wurden. Danach war gaschromatographisch kein Methanol mehr nachzuweisen.

Eine besonders empfindliche gaschromatographische head-space-Methode, durchgeführt vor und nach der Verseifung mit wässriger KOH, zeigte 45 ppm freies und 35 ppm eingebautes Methanol an.

Der Verkappungsgrad der endständigen OH-Gruppen liegt damit unter 0,05 %.

### Beispiel 6

### Natriumalkoholat katalysierte Methanolabspaltung aus dem Oligocarbonat aus Versuch 4

In den 5 L Kessel aus Versuch 5 wurden 4 L Oligocarbonat aus Versuch 4 vorgelegt und anschließend bei 120°C und 20 bis 40 mbar flüchtige Bestandteile über eine Kühlfalle abgezogen. Nach 3 Stunden war der Methanolgehalt auf 1000 ppm gesunken. Dimethylcarbonat war nicht mehr nachweisbar. Daraufhin wurde begonnen, bei Normaldruck 40 NL Stickstoff pro Stunde durch das Einleitrohr einzublasen. In 3 Stunden fiel der Methanolgehalt auf 450 ppm, worauf 2 Stunden bei 140°C 60 NL Stickstoff pro Stunde eingeleitet wurden. Danach war gaschromatographisch kein Methanol mehr nachzuweisen.

Eine besonders empfindliche gaschromatographische head-space-Methode, durchgeführt vor und nach der Verseifung mit wässriger KOH zeigte 40 ppm freies und 32 ppm eingebautes Methanol an.

Der Verkappungsgrad der endständigen OH-Gruppen liegt damit unter 0,05 %.

### Beispiele 7-9

### Titanalkoholat katalysierte Umesterung von DMC mit einem Oligomerengemisch hergestellt aus Hexamethylendiol und Caprolacton in einem Semibatch betriebenen Kessel mit 10 bödiger Gegenstromkolonne

Die Apparatur bestand aus einem 5 L-Planschlifftopf mit Blattrührer, Wellenbrechern, Einleitrohr, einer 10 bödigen ölbeheizten Glockenbodenkolonne mit Dephlegmator und einem Totalkondensator für das die Kolonne verlassende Gas. Die Kolonne hatte einen Flüssigkeits-hold-up von ca. 170 ml.

Kolonne und Kessel wurden auf 200°C aufgeheizt und mit Stickstoff vollständig inertisiert. Der Dephlegmator wurde auf 80°C thermostatisiert.

Eine äquimolare Mischung aus Hexamethylendiol und Caprolacton mit 1 % Titantetraisopropylat wurde hergestellt, dies entspricht einem Titangehalt von 0,14 %.

Dann wurde begonnen, pro Stunde 1 392 g 160°C warmes Produkt aus Hexamethylendiol und Caprolacton mit 0,14 % Titan auf den obersten Boden zu pumpen.

Nachdem 464 g Diol zugepumpt worden waren, wurde der Rührer in Betrieb genommen und begonnen, zusätzlich 540 g DMC pro Stunde durch das Tauchrohr in die Flüssigkeit am Boden des Kessels zu fördern.

Nach 10 Minuten wurde die Dosierung für weitere 70 Minuten auf 928 g DMC und 2 386 g Diol-Gemisch pro Stunde eingestellt.

Abschließend wird für 10 Minuten wieder mit den ursprünglichen Pumpraten dosiert.

Insgesamt wurden ca. 880 g Destillat in dieser Zeit erhalten, das aus ca. 85 % Methanol und 15 % Dimethylcarbonat besteht.

Der Sumpf enthält 1,93 % Methanol.

Dimethylcarbonat, Hexamethylendiol und Caprolacton sind nicht nachweisbar.

Aufgrund des unumgesetzten Dimethylcarbonates im Destillat wurden nochmals 223 g Dimethylcarbonat innerhalb von 25 Minuten nachdosiert, dabei entstehen nochmals 164 g eines Destillates aus 94 % Methanol und 5,6 % DMC.

Anschließend werden 200 NL Stickstoff pro Stunde durch das Einleitungsrohr in den Planschlifftopf geleitet. Nach 4 bis 5 Stunden Stickstoff einleiten werden noch 300 ppm eingebautes Methanol gefunden, die nach 6 bis 7 Stunden auf ca. 40 ppm gesunken sind, was einem Verkappungsgrad der endständigen OH-Gruppen von ca. 0,25 % entspricht.

Das entstandene aliphatische Oligocarbonat ist schwach gelblich gefärbt.

Werden 100 NL statt 200 NL Stickstoff durch das Tauchrohr geleitet, werden etwa 12 bis 14 Stunden benötigt, um den Gehalt an eingebautem Methanol auf ca. 40 ppm zu senken.

Bei 50 NL Stickstoff werden ca. 21 bis 22 Stunden benötigt.

### Beispiele 10-11

### Titanalkoholat katalysierte Umesterung von DMC mit einem Oligomerengemisch hergestellt aus Hexamethylendiol und Caprolacton in einem Semibatch betriebenen Kessel mit 10 bödiger Gegenstromkolonne mit anschließendem Inertgasstrippen unter vermindertem Druck

Die Apparatur, wie sie in Beispiel 7 beschrieben ist, wurde umgebaut, so dass die Kolonne durch Betätigung eines Ventiles vom Planschlifftopf getrennt und statt dessen über Kühlfallen eine Vakuumpumpe angeschlossen und der Druck geregelt werden kann. Der Stickstofffluss wurde über einen Massenflussregler eingestellt und konstant gehalten.

Die Versuchsbedingungen waren bis zum Zeitpunkt des Strippens mit Stickstoff wie in den Beispiel 7 bis 9 beschrieben.

Nach dem Abschluss des Einleitens von DMC wurde die Kolonne durch Schließen des Ventils abgetrennt und die Vakuumanlage, eingestellt auf konstante 150 mbar Saugdruck, angeschlossen.

Der Stickstoffstrom wurde ab diesem Zeitpunkt mit folgendem Stufengradienten über ein Tauchrohr eingeleitet: 0. bis 1. Stunde keine Stickstoffeinleitung, 1. bis 2. Stunde 2 NL/h, 2. bis 7. Stunde 20 NL/h, 7. bis 12. Stunde 40 NL/h und 12. bis 17. Stunde 90 NL/h.

Durch Begasung über ein einfaches Tauchrohr wurde nach 17 Stunden ein Gehalt an eingebautem Methanol von 66 ppm erreicht.

Begasung über eine 3 cm lange Glasfritte ergab einen Gehalt an eingebautem Methanol von 45 ppm bereits nach 12 Stunden.

### Beispiele 12-13

### Titanalkoholat katalysierte Umesterung von DMC mit einem Oligomerengemisch hergestellt aus Hexamethylendiol und Caprolacton in einem Semibatch betriebenen Kessel mit Begasungsrührer und 10 bödiger Gegenstromkolonne mit anschließendem Inertgasstrippen unter vermindertem Druck

Die Apparatur, wie sie in Beispiel 7 beschrieben ist, wurde umgebaut, so dass die Kolonne durch Betätigung eines Ventiles vom Planschlifftopf getrennt und statt dessen über Kühlfallen eine Vakuumpumpe angeschlossen und der Druck geregelt werden kann. Der Stickstofffluss wurde über einen Massenflussregler eingestellt und konstant gehalten.

Der Planschlifftopf wurde mit einem Begasungsrührer mit zwei Austrittsstellen für die Begasung des Kesselinhaltes ausgerüstet.

Das in die flüssige Phase eingetragene Gas konnte wahlweise aus dem Kessel selbst stammen oder als definiert eingestellter Gasstrom von außen eingeleitet sein.

Der Begasungsrührer lief mit ca. 850 Umdrehungen pro Minute.

Die Versuchsbedingungen waren bis zum Zeitpunkt des Strippens mit Stickstoff wie in den Beispielen 10 bis 11 beschrieben, mit der Ausnahme, dass der Begasungsrührer die Gasphase des Kessels sehr effizient in die Flüssigphase eintrug.

Durch den Einsatz des Begasungsrührers sank die Destillatmenge auf ca. 700 g und bestand zu ca. 95 % aus Methanol und 5 % aus Dimethylcarbonat.

Der Sumpf enthielt 2,16 % Methanol. Dimethylcarbonat, Hexamethylendiol und Caprolacton sind nicht nachweisbar.

Aufgrund des unumgesetzten Dimethylcarbonates im Destillat wurden nochmals 77 g Dimethylcarbonat innerhalb von 9 Minuten nachdosiert, dabei entstehen nochmals ca. 57 g eines Destillates aus 98,2 Methanol und 1,8 % DMC.

Nach dem Abschluss des Einleitens von DMC wurde die Kolonne durch Schließen des Ventils abgetrennt und die Vakuumanlage, eingestellt auf konstante 150 mbar Saugdruck, angeschlossen.

Der Stickstoffstrom wurde ab diesem Zeitpunkt mit folgendem Stufengradienten über das Tauchrohr eingeleitet: 0. bis 1. Stunde keine Stickstoffeinleitung, 2. bis 2. Stunde 2 NL/h und 2. bis 7. Stunde 20 NL/h.

Durch Begasung über ein einfaches Tauchrohr wurde durch den mitlaufenden Begasungsrührer schon nach 7 Stunden ein Gehalt an eingebautem Methanol von 68 ppm erreicht.

Erfolgte die Stickstoffeinleitung über den Begasungsrührer, der dann vom Gasraum des Kessels abgekoppelt war, wurde bereits nach 6 Stunden mit durchgehend 2 NL Stickstoff pro Stunde ein Gehalt an eingebautem Methanol von 40 ppm erreicht.

### Beispiel 14

### Vergleichsversuch mit Vakuumstrippen ohne Gasblasenerzeugung

Versuch 10 wurde wiederholt. Jedoch wurde nach dem Anlegen des Vakuums von 150 mbar ohne Einleiten von Stickstoff gerührt. Nach 8 Stunden war ein Restgehalt von ca. 4 000 ppm Methanol nachzuweisen. Nach 24 Stunden war dieser Methanol-gehalt auf ca. 1 000 ppm Methanol gesunken.

Es ist damit zu rechnen, dass die Raumzeitausbeute bei Vergrößerung des Ansatzes in einen technischen Maßstab von mehreren m³ nochmals deutlich sinkt.

## Patentansprüche

1. Verfahren zur Herstellung aliphatischer Oligocarbonate mit einem Verkappungsgrad der endständigen OH-Gruppen durch Methoxycarbonyl-Gruppen kleiner als 5 % durch Umsetzung von Dimethylcarbonat mit aliphatischen Diolen mit einem Umsatzgrad des eingesetzten Dimethylcarbonates größer 80 %, **dadurch gekennzeichnet, dass** die Umsetzung von Dimethylcarbonat und aliphatischen Diolen beschleunigt durch lösliche Katalysatoren in einer Gas-Flüssigkeits-Gegenstromapparatur durchgeführt wird, gefolgt von einer Oligomerisation unter Abspaltung von Methanol und Spuren Dimethylcarbonat in einer Apparatur die Gasblasen im Oligocarbonat erzeugt.

2. Verfahren nach Anspruch 1, **dadurch gekennzeichnet, dass** als Gas-Flüssigkeits-Gegenstromapparatur eine Glockenbodenkolonne mit 2 bis 20 Böden verwendet wird.

3. Verfahren nach Anspruch 1, **dadurch gekennzeichnet, dass** als Gas-Flüssigkeits-Gegenstromapparatur eine Blasensäulen-Kaskade mit 2 bis 8 Blasensäulen verwendet wird, wobei mehrere Blasensäulen ohne Einbauten zur Verhinderung von Rückvermischungen durch eine Kolonne mit Einbauten ersetzt werden können.

4. Verfahren nach Anspruch 1, **dadurch gekennzeichnet, dass** als Gas-Flüssigkeits-Gegenstromapparatur eine Kesselkaskade mit 2 bis 6 Kesseln verwendet wird, wobei die Kessel bevorzugt mit Begasungsrührern ausgestattet sind.

5. Verfahren nach Anspruch 1, **dadurch gekennzeichnet, dass** in der Gegenstromapparatur bei Temperaturen zwischen 100 und 250°C bei Drucken zwischen 0,8 und 8 bar gearbeitet wird.

6. Verfahren nach Anspruch 1, **dadurch gekennzeichnet, dass** als lösliche Katalysatoren die Hydroxide der Alkali- und Erdalkalimetalle und die Metallalkoholate von Metallen der I., II., III. und IV. Hauptgruppe, der III. und IV. Nebengruppe oder aus der Gruppe der Seltenerden eingesetzt werden.

7. Verfahren nach Anspruch 1, **dadurch gekennzeichnet, dass** als Apparatur die Gasblasen im Oligocarbonat erzeugt, eine Blasensäulen-Kaskade oder eine Kesselkaskade mit Begasungsrührern eingesetzt wird.

8. Verfahren nach Anspruch 6, **dadurch gekennzeichnet, dass** in der Apparatur die Gasblasen im Oligocarbonat erzeugt, bei Temperaturen zwischen 150 und 250°C und bei Drucken zwischen 0,01 und 1 bar gearbeitet wird.

9. Verfahren nach Anspruch 1, **dadurch gekennzeichnet, dass** das Verfahren als Semibatch-Verfahren durchgeführt wird mit einem großen Kessel, bevorzugt mit Begasungsrührer, in den das Dimethylcarbonat dosiert wird und in dem abschließend die Demethoxylierung stattfindet sowie mit aufgesetzter Glockenbodenkolonne, kleiner Kesselkaskade oder Blasensäulenkaskade, in der die Gegenstromumesterung stattfindet, wobei das Flüssigkeitsvolumen des Gegenstromanlagenteils zwischen 0,5 und 50 % des Kesselvolumens des großen Kessels beträgt.

10. Verfahren nach Anspruch 1, **dadurch gekennzeichnet, dass** Oligocarbonatdiole der Formel (I) 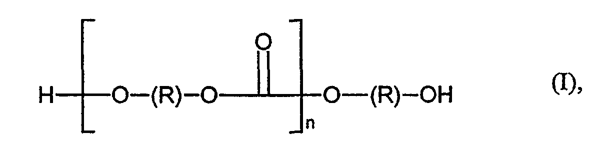 in welcher
(R) für aliphatische Reste mit einer Kohlenstoffzahl von 3 bis 50,
die gegebenenfalls Ester, Ether, Amid und Nitril-Funktionen enthalten können, bedeutet,
und n für eine ganze Zahl von 2 bis 30 steht,
eingesetzt werden,

11. Verfahren nach Anspruch 1, **dadurch gekennzeichnet, dass** als Diole Pentamethylendiol, Hexamethylendiol, Neopentylglykol, Cyclohexandimethanol und die Umsetzungsprodukte aus Caprolacton mit den oben genannten Alkoholen und Mischungen dieser Diole benutzt werden.

12. Verfahren nach Anspruch 1, **dadurch gekennzeichnet, dass** die Gasblasen in der gasblasenerzeugenden Apparatur durch Einleiten von inerten Gasen wie Stickstoff, Argon, Methan, Ethan, Propan, Butan, Dimethylether, trockenem Erdgas oder trockenem Wasserstoff erzeugt werden, wobei der das Oligocarbonat verlassende Methanol und Dimethylcarbonat-haltige Gasstrom teilweise zur Sättigung erneut dem Oligocarbonat zugeführt werden kann.

13. Verfahren nach Anspruch 1, **dadurch gekennzeichnet, dass** die Gasblasen in der gasblasenerzeugenden Apparatur durch Einleiten von inerten leichtsiedenden Flüssigkeiten wie Pentan, Cyclopentan, Hexan, Cyclohexan, Petrolether, Diethylether oder Methyl-tert.-Butylether erzeugt werden, wobei die Stoffe flüssig oder gasförmig eingeleitet werden können und der das Oligocarbonat verlassende Methanol und Dimethylcarbonat-haltige Gasstrom teilweise zur Sättigung erneut dem Oligocarbonat zugeführt werden kann.

## Claims

1. Process for the preparation of aliphatic oligocarbonates with a degree of capping of the terminal OH groups by methoxycarbonyl groups of less than 5%, by reacting dimethyl carbonate with aliphatic diols, with a degree of conversion of the initial dimethyl carbonate of more than 80%, **characterized in that** the reaction of dimethyl carbonate and aliphatic diols, accelerated by soluble catalysts, is carried out in a gas-liquid countercurrent apparatus, followed by oligomerization, with the elimination of methanol and traces of dimethyl carbonate, in an apparatus which generates gas bubbles in the oligocarbonate.

2. Process according to claim 1, **characterized in that** the gas-liquid countercurrent apparatus used is a bubble-cap column with 2 to 20 plates.

3. Process according to claim 1, **characterized in that** the gas-liquid countercurrent apparatus used is a bubble column cascade with 2 to 8 bubble columns, it being possible for several bubble columns without baffles to be replaced by one column with baffles in order to prevent back-mixing.

4. Process according to claim 1, **characterized in that** the gas-liquid countercurrent apparatus used is a tank cascade with 2 to 6 tanks, which are preferably equipped with gas distribution stirrers.

5. Process according to claim 1, **characterized in that** the countercurrent apparatus is operated at temperatures of between 100 and 250°C and at pressures of between 0.8 and 8 bar.

6. Process according to claim 1, **characterized in that** the soluble catalysts used are alkali metal and alkaline earth metal hydroxides and alcoholates of metals of main groups I, II, III and IV, subgroups III and IV or the rare earth group.

7. Process according to claim 1, **characterized in that** a bubble column cascade or a tank cascade with gas distribution stirrers is used as the apparatus which generates gas bubbles in the oligocarbonate.

8. Process according to claim 6, **characterized in that** the apparatus which generates gas bubbles in the oligocarbonate is operated at temperatures of between 150 and 250°C and at pressures of between 0.01 and 1 bar.

9. Process according to claim 1, **characterized in that** it is carried out as a semibatch process with a large tank, preferably with a gas distribution stirrer, into which the dimethyl carbonate is metered and in which the demethoxylation ultimately takes place, and with an attached bubble-cap column, small tank cascade or bubble column cascade, in which the countercurrent transesterification takes place, the liquid volume of the countercurrent part of the plant being between 0.5 and 50% of the volume of the large tank.

10. Process according to claim 1, **characterized in that** oligocarbonatediols of formula (I): 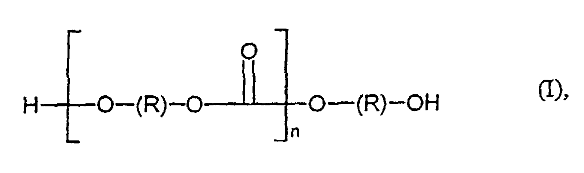 are used in which:
(R) represents aliphatic radicals with a carbon number of 3 to 50, which can optionally contain ester, ether, amide and nitrile groups, and
n is an integer from 2 to 30.

11. Process according to claim 1, **characterized in that** the diols used are pentamethylenediol, hexamethylenediol, neopentyl glycol, cyclohexanedimethanol, the reaction products of caprolactone with the above-mentioned alcohols, and mixtures of these diols.

12. Process according to claim 1, **characterized in that** the gas bubbles in the apparatus which generates gas bubbles are produced by introducing inert gases such as nitrogen, argon, methane, ethane, propane, butane, dimethyl ether, dry natural gas or dry hydrogen, it being possible for part of the gas stream leaving the oligocarbonate, and containing methanol and dimethyl carbonate, to be recycled into the oligocarbonate in order to saturate it.

13. Process according to claim 1, **characterized in that** the gas bubbles in the apparatus which generates gas bubbles are produced by introducing inert low-boiling liquids like pentane, cyclopentane, hexane, cyclohexane, petroleum ether, diethyl ether or methyl tert-butyl ether, it being possible for the substances to be introduced in liquid or gaseous form and for part of the gas stream leaving the oligocarbonate, and containing methanol and dimethyl carbonate, to be recycled into the oligocarbonate in order to saturate it.

## Revendications

1. Procédé pour la préparation d'oligocarbonates aliphatiques, dont les groupes terminaux OH possèdent un degré de blocage via des groupes méthoxycarbonyle inférieur à 5 %, par mise en réaction de carbonate de diméthyle avec des diols aliphatiques, avec un degré de transformation du carbonate de diméthyle mis en oeuvre supérieur à 80 %, **caractérisé en ce qu'**on effectue la mise en réaction du diméthylcarbonate et des diols aliphatiques, accélérée à l'intervention de catalyseurs solubles, dans un appareil d'écoulement de gaz-liquide travaillant à contre-courant, et on procède ensuite à une oligomérisation avec élimination du méthanol et des traces de carbonate de diméthyle dans un appareil qui génère des bulles de gaz dans l'oligocarbonate.

2. Procédé selon la revendication 1, **caractérisé en ce qu'**on utilise, à titre d'appareil d'écoulement de gaz-liquide travaillant à contre-courant, une colonne du type à plateaux à cloches comprenant de 2 à 20 plateaux.

3. Procédé selon la revendication 1, **caractérisé en ce qu'**on utilise, à titre d'appareil d'écoulement de gaz-liquide travaillant à contre-courant, une cascade de colonnes à bulles comprenant de 2 à 8 colonnes à bulles, plusieurs colonnes à bulles exemptes de chicanes pour empêcher des remélangeages pouvant être remplacées par une colonne équipée de chicanes.

4. Procédé selon la revendication 1, **caractérisé en ce qu'**on utilise, à titre d'appareil d'écoulement de gaz-liquide travaillant à contre-courant, une cascade de cuves comprenant de 2 à 6 cuves, les cuves étant de préférence équipées d'agitateurs de gazéification.

5. Procédé selon la revendication 1, **caractérisé en ce que**, dans l'appareil du type à contre-courant on travaille à des températures entre 100 et 250 °C sous des pressions entre 0,8 et 8 bar.

6. Procédé selon la revendication 1, **caractérisé en ce qu'**on met en oeuvre, à titre de catalyseurs solubles, les hydroxydes de métaux alcalins et de métaux alcalino-terreux, ainsi que des alcoolates métalliques des métaux des groupes principaux I, II, III et IV, des sous-groupes III et IV ou encore du groupe des terres rares.

7. Procédé selon la revendication 1, **caractérisé en ce qu'**on met en oeuvre, à titre d'appareil qui génère des bulles de gaz dans l'oligocarbonate, une cascade de colonnes à bulles ou une cascade de cuves équipées d'agitateurs de gazéification.

8. Procédé selon la revendication 6, **caractérisé en ce que**, dans l'appareil qui génère des bulles de gaz dans l'oligocarbonate, on travaille à des températures entre 150 et 250 °C et sous des pressions entre 0,01 et 1 bar.

9. Procédé selon la revendication 1, **caractérisé en ce qu'**on effectue le procédé sous la forme d'un procédé en semi-continu avec une cuve de grande dimension, de préférence équipée d'agitateurs de gazéification, dans laquelle on introduit par dosage le carbonate de diméthyle et dans laquelle a lieu, pour terminer, la déméthoxylation, ainsi qu'avec une colonne du type à plateaux à cloches, une cascade de cuves ou une cascade de colonnes à bulles disposée par-dessus de plus petites dimensions, dans laquelle a lieu la transestérification à contre-courant, le volume de liquide de la partie d'installation à contre-courant représentant entre 0,5 et 50 % du volume de la cuve de grande dimension.

10. Procédé selon la revendication 1, **caractérisé en ce qu'**on met en oeuvre des oligocarbonate-diols répondant à la formule (I) 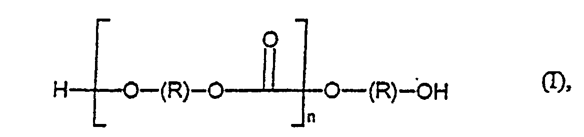 dans laquelle
(R) représente des radicaux aliphatiques contenant de 3 à 50 atomes de carbone, qui peuvent contenir, le cas échéant, des fonctions esters, éthers, amides et nitriles,
et n représente un nombre entier de 2 à 30.

11. Procédé selon la revendication 1, **caractérisé en ce qu'**on utilise à titre de diols, le pentaméthylène-diol, l'hexaméthylène-diol, le néopentylglycol, le cyclohexane-diméthanol et des produits réactionnels de caprolactone avec les alcools mentionnés ci-dessus, ainsi que des mélanges de ces diols.

12. Procédé selon la revendication 1, **caractérisé en ce qu'**on génère les bulles de gaz dans l'appareil destiné à cet effet, en faisant passer des gaz inertes tels que l'azote, l'argon, le méthane, l'éthane, le propane, le butane, l'éther diméthylique, le gaz naturel sec ou de l'hydrogène sec, le courant de gaz contenant du méthanol et du carbonate de diméthyle quittant l'oligocarbonate pouvant être renvoyé en partie à l'oligocarbonate à des fins de saturation.

13. Procédé selon la revendication 1, **caractérisé en ce qu'**on génère les bulles de gaz dans l'appareil destiné à cet effet, en faisant passer des liquides inertes à bas point d'ébullition tels que le pentane, le cyclopentane, l'hexane, le cyclohexane, l'éther de pétrole, l'éther diéthylique ou l'éther méthyl-tert.-butylique, les substances pouvant être introduites sous forme liquide ou sous forme gazeuse, et le courant de gaz contenant du méthanol et du carbonate de diméthyle quittant l'oligo-carbonate pouvant être renvoyé en partie à l'oligo-carbonate à des fins de saturation.
